# EUROPEAN PATENT SPECIFICATION

(11) **EP 3 763 327 B1**
(45) Date of publication and mention of the grant of the patent: **15.05.2024**
(21) Application number: 18909066.5
(22) Date of filing: 11.12.2018
(51) Int. Cl.: A61F 2/07, A61F 2/852, A61F 2/06

(54) **AORTA TREATMENT DEVICE**
AORTENBEHANDLUNGSVORRICHTUNG
DISPOSITIF DE TRAITEMENT D'AORTE

(30) Priority: 09.03.2018 JP 2018043445
(43) Date of publication of application: 13.01.2021
(73) Proprietor: Japan Lifeline Co., Ltd., Tokyo 140-0002 (JP)
(72) Inventor: KOTANI Chikara, Tokyo 140-0002 (JP); SAKAI Masamune, Tokyo 140-0002 (JP)
(74) Representative: Grünecker Patent- und Rechtsanwälte PartG mbB
(86) International application number: PCT/JP2018/045473
(87) International publication number: WO 2019/171694

(56) References cited:
- EP-A2- 3 578 134
- WO-A1-2018/032358
- JP-A- 2004 533 868
- JP-A- 2007 501 091
- JP-A- 2012 086 004
- JP-A- 2018 083 082
- US-A1- 2004 082 989
- US-A1- 2006 184 229
- US-A1- 2007 038 288
- US-A1- 2008 195 191
- US-A1- 2011 270 378
- US-A1- 2013 123 902
- US-A1- 2015 039 074

## Description

### Technical Field

The present invention relates to a treatment device for treating a disease in a thoracic aorta.

### Background Art

As a method for treating a disease in a thoracic aorta, such as an aortic aneurysm or an aortic dissection, artificial blood vessel replacement surgery and stent graft insertion are performed.

Here, as an artificial blood vessel used for artificial blood vessel replacement surgery of replacing an aortic arch, a branched artificial blood vessel including four side-branch tubes is known (see PTL 1). As a stent graft used for stent graft insertion, various types are known, and, for example, a stent graft having a structure that can improve utility for a treatment using an OSG (Open Stent Graft) method has been proposed by the present applicant (see PTL 2).

Recently, in order to improve the efficiency of manipulation, an aorta treatment device having a structure in which a branched artificial blood vessel, including four side-branch tubes, and a stent graft are sutured and integrated has been introduced.

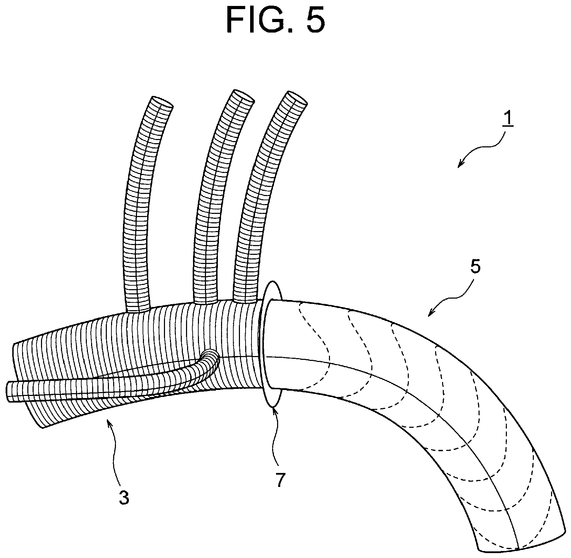

Fig. 5 is a view illustrating such a treatment device, and the treatment device 1 includes a branched artificial blood vessel portion 3, a stent graft portion 5 sutured to the distal side thereof, and a flange-shaped collar 7 attached to the sutured part of the branched artificial blood vessel portion 3 and the stent graft portion 5.

The treatment device 1 is attached by using a delivery system (not shown) as follows. First, from a disjoined part after an aortic arch has been excised, the stent graft portion 5 in a reduced-diameter state is inserted into a distal aorta (descending part of aorta), which is a living blood vessel, and the treatment device 1 is disposed so as to block an opening in a proximal end part (the disjoined part) of the distal aorta with the flange-shaped collar 7.

Next, the stent graft portion 5 is increased in diameter and thus brought into close contact with an inner wall of the distal aorta, the size of the collar 7 is adjusted by cutting off a part thereof as necessary, and the distal aorta and the branched artificial blood vessel portion 3 are anastomosed by suturing the collar 7, which has been cut to an appropriate size, and the proximal end part of the distal aorta. Subsequently, by suturing a proximal end part of the branched artificial blood vessel portion 3 and a proximal aorta (ascending part of aorta), which is a living blood vessel, the branched artificial blood vessel portion 3 and the proximal aorta are anastomosed. Further relevant prior art is described in WO 2018/032358 A1, EP 3 578 134 A2 and US 2011/270378 A1. Herein EP 3 578 134 A2 is a post-published document.

### Citation List

### Patent Literature

PTL 1: Japanese Unexamined Patent Application Publication No. 7-308330
PTL 2: Japanese Unexamined Patent Application Publication No. 2017-23464

### Summary of Invention

### Technical Problem

However, the treatment device described above has a problem in that it is extremely difficult to perform the manipulation of aligning the position of the proximal end part of the distal aorta, whose end surface shape differs among patients, with the position of the flange-shaped collar, and suturing these while adjusting the shapes and the sizes thereof; and tension tends to remain after suturing.

Moreover, the inside diameter of the distal aorta, which includes a lesion such as an aortic aneurysm or an aorta dissection, has individual difference among patients, and there is a case where it is not possible to bring the stent graft portion into sufficiently close contact with the inner wall of the distal aorta by using the treatment device described above.

The present invention has been made based on the circumstances described above.

An object of the present invention is to provide an aorta treatment device that can replace an aortic arch with a branched artificial blood vessel portion, place a stent graft portion in a distal aorta, and anastomose the branched artificial blood vessel portion with the distal aorta easily compared with an existing device.

Another object of the present invention is to provide an aorta treatment device including a stent graft portion that can be brought into sufficiently close contact with an inner wall of a distal aorta, with which close contact of a stent graft portion of an existing treatment device is insufficient.

### Solution to Problem

In particular it is provided an aorta treatment device, having the feature defined in claim 1. Further preferred embodiments are defined in the dependent claims.
(1) An aorta treatment device according to the present invention includes: a branched artificial blood vessel portion including a main tube and at least one side tube that branches off from the main tube; a stent graft portion coupled to a distal side of the branched artificial blood vessel portion; and a cuff portion that is formed so as to extend from a distal end of the branched artificial blood vessel portion toward the distal side, that is evertable so as to enter a state in which the cuff portion extends from the distal end of the branched artificial blood vessel portion toward a proximal side, and that has a cylindrical shape or a skirt-like shape.

With the treatment device having such a configuration, it is possible to anastomose a distal aorta and the branched artificial blood vessel portion by: everting the cuff portion; inserting the stent graft portion in a reduced-diameter state into the distal aorta until the position of an open end of the everted cuff portion substantially coincides with the position of a proximal end of the distal aorta; increasing the stent graft portion in diameter and placing the stent graft portion in the distal aorta; and then suturing the proximal end part of the distal aorta and the everted cuff portion.

Here, because the proximal end part of the distal aorta and the cuff portion can be disposed in substantially coaxial cylindrical shapes when suturing these, it is significantly easy to perform manipulation of suturing these, compared with an existing treatment device including a flange-shaped collar, and tension does not remain after suturing.

(2) In the aorta treatment device according to the present invention, preferably, the branched artificial blood vessel portion includes four side tubes that branch off from the main tube.

(3) In the aorta treatment device according to the present invention, preferably, the cuff portion is formed from a material that is the same as a material of the branched artificial blood vessel portion so as to be continuous with the distal end of the branched artificial blood vessel portion (that is, the branched artificial blood vessel portion and the cuff portion are formed from a common tubular knit fabric), and the stent graft portion is coupled to the branched artificial blood vessel portion by being sutured to the distal end of the branched artificial blood vessel portion.

With the treatment device having such a configuration, the integrity of the branched artificial blood vessel portion with the cuff portion can be further improved.

(4) In the aorta treatment device according to the present invention, preferably, a ratio of an inside diameter of the stent graft portion to an inside diameter of the main tube of the branched artificial blood vessel portion is 1.01 to 1.5.

With the treatment device having such a configuration, because the inside diameter ratio is 1.01 or higher, the stent graft portion can be brought into sufficiently close contact with an inner wall of the distal aorta, with which close contact of a stent graft portion has been insufficient with an existing treatment device.

(5) In the aorta treatment device according to the present invention, the cuff portion has a skirt-like shape such that a length thereof is 5 to 30 mm and a ratio of an inside diameter of an open end to an inside diameter of a base end is 1.05 to 1.3.

With the treatment device having such a configuration, due to the skirt-like shape such that the inside diameter ratio is 1.3 or lower, suturing with the proximal end part of the distal aorta can be more easily performed.

Moreover, because the inside diameter ratio is 1.05 or higher, suturing can be easily performed even if the shape (size) of the proximal end of the distal aorta has enlarged. Furthermore, an operation of everting the cuff portion is also easy.

(6) In the aorta treatment device according to the present invention, preferably, the branched artificial blood vessel portion and the cuff portion are treated with a coating for preventing leakage of blood, and the stent graft portion is not treated with the coating.

With the treatment device having such a configuration, leakage of blood from the branched artificial blood vessel portion and the cuff portion, which are treated with the coating, can be prevented; and the stent graft portion, which is not treated with the coating, is flexible and thus can be easily reduced in diameter, can properly fit with a blood vessel, and has high compatibility with a living tissue. Moreover, there is no negative effect on a diameter-increasing operation after being stored in a reduced-diameter state for a long period, and storage stability is high.

### Advantageous Effects of Invention

With the treatment device according to the present invention, it is possible to replace an aortic arch with a branched artificial blood vessel portion, place a stent graft portion in a distal aorta, and anastomose the branched artificial blood vessel portion and the distal aorta easily compared with an existing treatment device.

With the treatment device according to the present invention including the stent graft portion having an inside diameter that is 1.01 times the inside diameter of the main tube of the branched artificial blood vessel portion or larger, the stent graft portion of the treatment device according to the present invention can be brought into sufficiently close contact with an inner wall of the distal aorta, with which a stent graft portion of an existing treatment device has not been capable of being brought into sufficiently close contact.

### Brief Description of Drawings

[Fig. 1A] Fig. 1A is a view of an aorta treatment device according to an embodiment of the present invention, illustrating a state in which a cuff portion is not everted.
[Fig. 1B] Fig. 1B is a view of the aorta treatment device according to the embodiment of the present invention, illustrating a state in which the cuff portion is everted.
[Fig. 2] Fig. 2 is a view illustrating a state in which the aorta treatment device shown in Fig. 1 is in use.
[Fig. 3] Fig. 3 is a sectional view taken along line III-III of Fig. 2.
[Fig. 4A] Fig. 4A is a view of an aorta treatment device according to another embodiment of the present invention, illustrating a state in which a cuff portion is not everted.
[Fig. 4B] Fig. 4B is a view of the aorta treatment device according to the other embodiment of the present invention, illustrating a state in which the cuff portion is everted.
[Fig. 5] Fig. 5 is a perspective view of an existing aorta treatment device.

### Description of Embodiments

### <First Embodiment>

Hereinafter, an aorta treatment device according to an embodiment of the present invention will be described in detail.

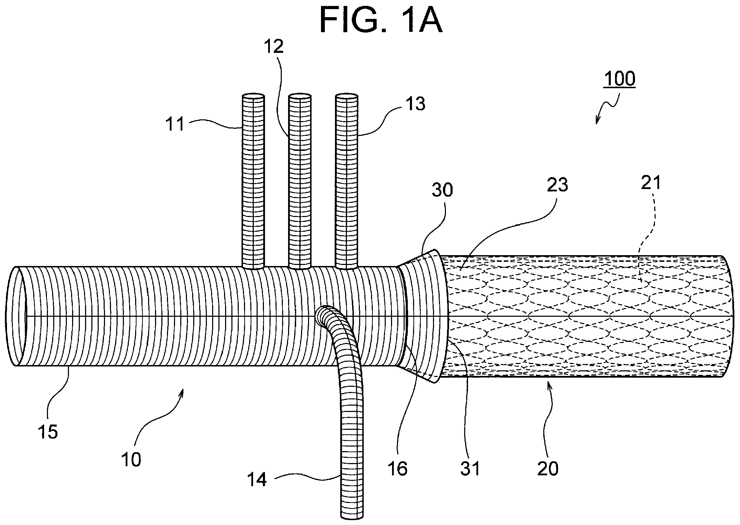

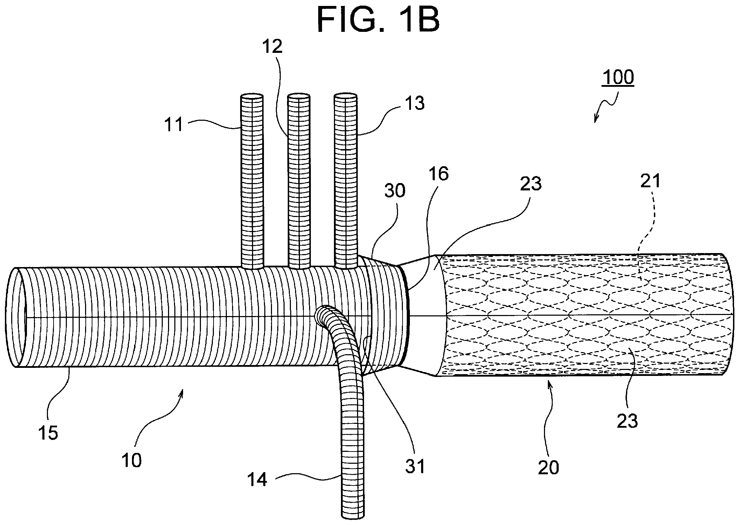

An aorta treatment device 100 according to the present embodiment, which is illustrated in Fig. 1 (Fig. 1A and Fig. 1B), includes: a branched artificial blood vessel portion 10 including four side tubes 11 to 14 that branch off from a main tube 15; a stent graft portion 20 coupled to a distal side of the branched artificial blood vessel portion 10 by being sutured to a distal end 16 of the branched artificial blood vessel portion 10; and a cuff portion 30 that is formed so as to extend from the distal end 16 of the branched artificial blood vessel portion 10 toward the distal side, that enters a state in which the cuff portion 30 extends from the distal end 16 of the branched artificial blood vessel portion 10 toward the proximal side by being everted, and that has an evertable skirt-like shape.

The branched artificial blood vessel portion 10 of the treatment device 100 according to the present embodiment includes the main tube 15 and the four side tubes 11 to 14 that branch off from the main tube 15.

The main tube 15 and the side tubes 11 to 14 are made from a tubular knit fabric.

The main tube 15 and the side tubes 11 to 14 have ribs formed therein, are durable to extension/contraction and bending, resistant to kinking, and easily conformable to the shape of a blood vessel of a human body.

The length (free length) of the main tube 15 is preferably 100 to 400 mm, and a preferable example is 210 mm.

The inside diameter of the main tube 15 is preferably 16 to 36 mm, and a preferable example is 26 mm.

Among the side tubes 11 to 14 that branch off from the main tube 15, the three side tube 11 to 13 form one group, and the side tube 14 is disposed so as to be displaced from this group.

The length of the side tubes 11 to 14 is preferably 50 to 300 mm, and a preferable example is 210 mm.

The inside diameter of the side tube 11 is preferably 5 to 14 mm, and a preferable example is 11 mm.

The inside diameter of the side tubes 12 and 13 is preferably 5 to 12 mm, and a preferable example of each is 9 mm.

The inside diameter of the side tube 14 is preferably 8 to 12 mm, and a preferable example is 9 mm.

As a tubular knit fabric from which the branched artificial blood vessel portion 10 (the main tube 15 and the side tubes 11 to 14) is made, a tubular item made of a fabric or a knit of thermoplastic resin fiber may be used, and a plain weave fabric of thermoplastic resin fiber may be preferably used.

The wall thickness of the tubular knit fabric is preferably 1 mm or smaller, and more preferably 0.3 to 0.7 mm.

Examples of a thermoplastic resin that forms the thermoplastic resin fiber include: polyolefin such as polyethylene, polypropylene, or an ethylene-α-olefin copolymer; polyamide; polyurethane; polyester such as polyethylene terephthalate, polybutylene terephthalate, polycyclohexane terephthalate, or polyethylene-2,6-naphtharate; a fluorocarbon resin such as PTFE or ETFE; and the like. Among these, polyester such as polyethylene terephthalate and a fluorocarbon resin such as PTFE or ETFE, each of which is chemically stable, highly durable, and unlikely to cause a tissue reaction, are preferable; and polyester having a weight-average molecular weight of 10000 to 200000, in particular, a weight-average molecular weight of 15000 to 100000 is particularly preferable.

The branched artificial blood vessel portion 10 is treated with a coating of collagen, gelatin, or the like, and thus leakage of blood from the branched artificial blood vessel portion 10 can be prevented.

The stent graft portion 20 of the treatment device 100 according to the present embodiment includes a selfexpandable stent 21 that can be held in a reduced-diameter state and a graft 23 that covers an outer periphery of the stent 21.

Although the stent graft portion 20 is illustrated in an increased-diameter state in Fig. 1 (Fig. 1A and Fig. 1B), until the diameter is increased in a living blood vessel, the stent graft portion 20 is attached to a delivery shaft (not shown) in a reduced-diameter state.

The length of the stent graft portion 20 is preferably 60 to 210 mm, and a preferable example is 110 mm.

As illustrated in Fig. 1 (Fig. 1A and Fig. 1B), the diameter of the stent graft portion 20 in an increased-diameter state is larger than the diameter of the main tube 15 of the branched artificial blood vessel portion 10.

Here, the inside diameter of the stent graft portion 20 is preferably 16 to 39 mm, and a preferable example is 30 mm.

The ratio of the inside diameter of the stent graft portion 20 to the inside diameter of the main tube 15 of the branched artificial blood vessel portion 10 is preferably 1.01 to 1.5, and a preferable example is 1.15 (30 mm/26 mm).

When the inside diameter of the stent graft portion 20 is 1.01 times the inside diameter of the main tube 15 or larger, the stent graft portion 20 can be brought into sufficiently close contact with an inner wall of the distal aorta, with which close contact of a stent graft portion has been insufficient with an existing treatment device.

The structure of the stent 21 of the stent graft portion 20 is not particularly limited, and examples of the structure include: a tubular structure made of a zig-zag wire material; a knit, fabric, or braid of one or a plurality of wire materials; a tubular structure in which a plurality of these are combined; a tubular structure formed by, for example, laser processing a plate-shaped or tubular structure made of a metal; and the like.

As a wire material and the material of a tubular metal structure of the stent 21, a metal wire material of stainless steel, tantalum, titanium, platina, gold, tungsten, a shape memory alloy of Ni-Ti, Cu-Al-Ni, Cu-Zn-Al, or the like may be used; and the surface thereof may be covered with, for example, gold, platinum, or the like by means of plating or the like.

The diameter of a wire material of the stent 21 is not particularly limited, and is preferably 0.08 to 1 mm.

The thickness of a tubular metal structure of the stent 21 is not particularly limited, and is preferably 0.08 to 1 mm.

As the graft 23 of the stent graft portion 20, an item formed from a thermoplastic resin to have a cylindrical shape by performing a forming method such as extrusion or blow molding, a knit fabric of thermoplastic resin fiber having a cylindrical shape, a non-woven fabric of a thermoplastic resin having a cylindrical shape, a thermoplastic resin sheet or a porous sheet having a cylindrical shape, or the like may be used.

Ribs are not formed in the graft 23, and thus the graft 23 can be brought into sufficiently close contact with a blood vessel inner wall.

Examples of a thermoplastic resin material of the graft 23 include thermoplastic resins that have been shown above as examples of the material of thermoplastic resin fiber of the branched artificial blood vessel portion 10 (tubular knit fabric).

The stent graft portion 20 (the graft 23) is not treated with the coating of an antithrombogenic material, with which the branched artificial blood vessel portion 10 is treated.

The stent graft portion 20, which is not treated with a coating, is flexible and thus can be easily reduced in diameter, can properly fit to a blood vessel, and has high compatibility with a living tissue. Moreover, there is no negative effect on a diameter-increasing operation after being stored in a reduced-diameter state for a long period.

The cuff portion 30 of the treatment device 100 according to the present embodiment is formed from a material (knit fabric) that is the same as the material of the branched artificial blood vessel portion 10 so as to be continuous with the distal end 16 of the branched artificial blood vessel portion 10.

That is, the branched artificial blood vessel portion 10 and the cuff portion 30 are formed from one tubular knit fabric. Moreover, the stent graft portion 20 is coupled to the branched artificial blood vessel portion 10 by being sutured to the distal end 16 of the branched artificial blood vessel portion 10.

Thus, the treatment device 100 has high integrity between the branched artificial blood vessel portion 10 and the cuff portion 30, and can reliably avoid leakage of blood from a space between the branched artificial blood vessel portion 10 and the cuff portion 30.

As illustrated in Fig. 1A, in a non-everted state, the cuff portion 30 of the treatment device 100 extends from the distal end 16 of the branched artificial blood vessel portion 10 toward the distal side so as to cover an outer periphery of a proximal end part of the stent graft portion 20, and an open end 31 of the cuff portion 30 is positioned on the distal side of the distal end 16 of the branched artificial blood vessel portion 10.

The cuff portion 30 has a skirt-like shape such that the inside diameter of the open end 31 is larger than the inside diameter of the base end thereof (the distal end 16 of the branched artificial blood vessel portion 10).

Here, the length of the cuff portion 30 (the increased-diameter part of the tubular knit fabric) is preferably 5 to 30 mm, and a preferable example is 15 mm.

The inside diameter of the cuff portion 30 at the base end is the same as the inside diameter of the main tube 15 of the branched artificial blood vessel portion 10.

The inside diameter of the cuff portion 30 at the open end 31 is preferably 16 to 47 mm, and a preferable example is 28 mm.

The ratio of the inside diameter of the open end 31 to the inside diameter of the base end of the cuff portion 30 (the inside diameter of the main tube 15 of the branched artificial blood vessel portion 10) is preferably 1.05 to 1.3, and a preferable example is 1.08 (28 mm/26 mm).

Because the inside diameter ratio is 1.05 or higher, suturing can be easily performed even if the shape (size) of the proximal end of the distal aorta has enlarged. Moreover, an operation of everting the cuff portion can also be easily performed.

Although it is difficult to perform suturing with a proximal end part of the distal aorta if the inside diameter ratio is excessively high (the inside diameter of the open end 31 >> the inside diameter of the base end), when the inside diameter ratio is 1.3 or lower, suturing with the proximal end part of the distal aorta can be easily performed.

The cuff portion 30 can be everted so as to be turned inside out (so that an inner periphery and an outer periphery are reversed).

Fig. 1B illustrates a state in which the cuff portion 30 is everted, the everted cuff portion 30 extends from the distal end 16 of the branched artificial blood vessel portion 10 toward the proximal side, and the open end 31 of the cuff portion 30 is positioned on the proximal side of the distal end 16 of the branched artificial blood vessel portion 10.

The cuff portion 30 is treated with a coating of collagen, gelatin, or the like, and thus leakage of blood from the cuff portion 30 can be prevented.

The treatment device 100 according to the present embodiment is attached by using a delivery system (not shown) as follows.

First, in a state in which the cuff portion 30 is everted, the stent graft portion 20 in a reduced-diameter state is inserted from a disjoined part of an aortic arch into a distal aorta (descending part of aorta), and the position of the open end 31 of the everted cuff portion 30 is adjusted to substantially coincide with the position of the proximal end of the distal aorta. Next, the stent graft portion 20 is increased in diameter and placed in the distal aorta.

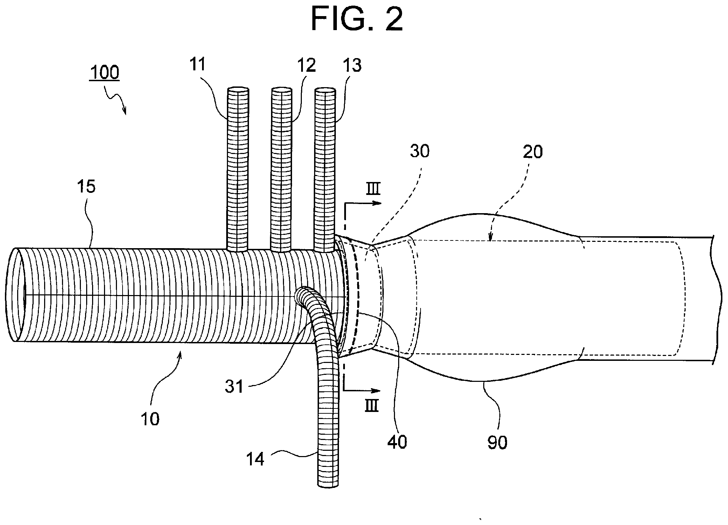

Subsequently, as illustrated in Fig. 2, a distal aorta 90 and the branched artificial blood vessel portion 10 are anastomosed by suturing the proximal end part of the distal 0aorta 90 and the cuff portion 30 by using a suture thread 40.

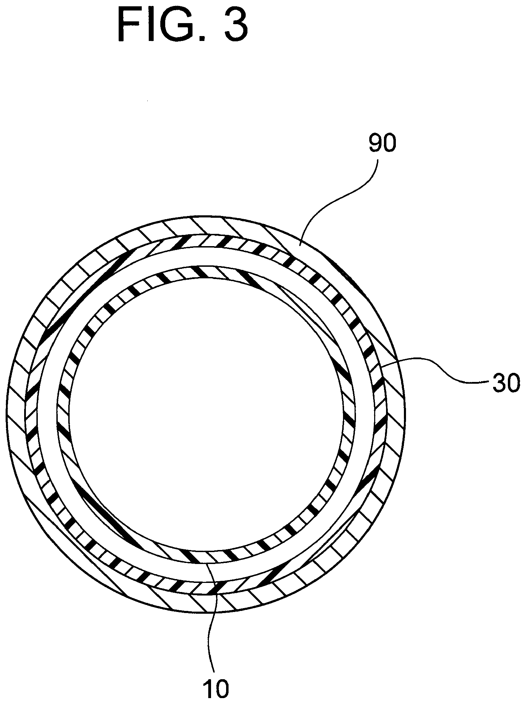

As illustrated in Fig. 2 and Fig. 3, because the proximal end part of the distal aorta 90 and the cuff portion 30 can be disposed in substantially coaxial cylindrical shapes when suturing these, it is not necessary to perform a cumbersome operation, such as adjustment of the size of a collar, which has been performed when using an existing treatment device, these can be easily sutured, and tension does not remain after suturing.

Subsequently, by suturing the proximal end part of the branched artificial blood vessel portion 10 and the proximal aorta (ascending part of aorta), the branched artificial blood vessel portion 10 and the proximal aorta are anastomosed.

### <Second Embodiment>

Next, an artificial blood vessel according to another embodiment of the present invention will be described in detail.

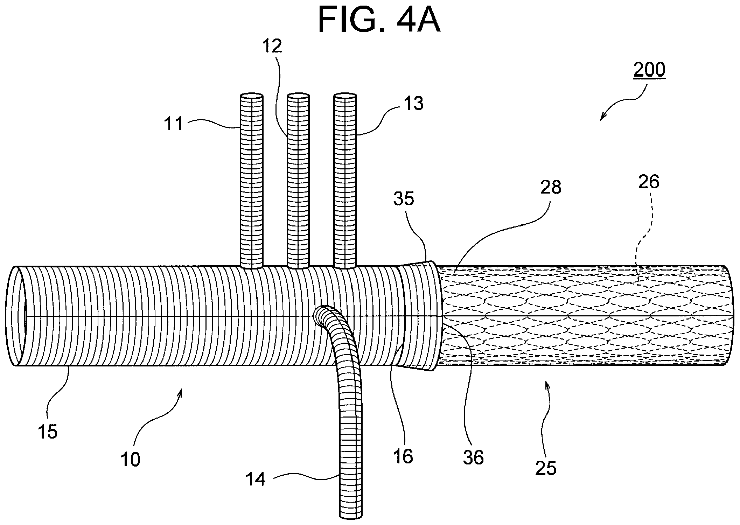

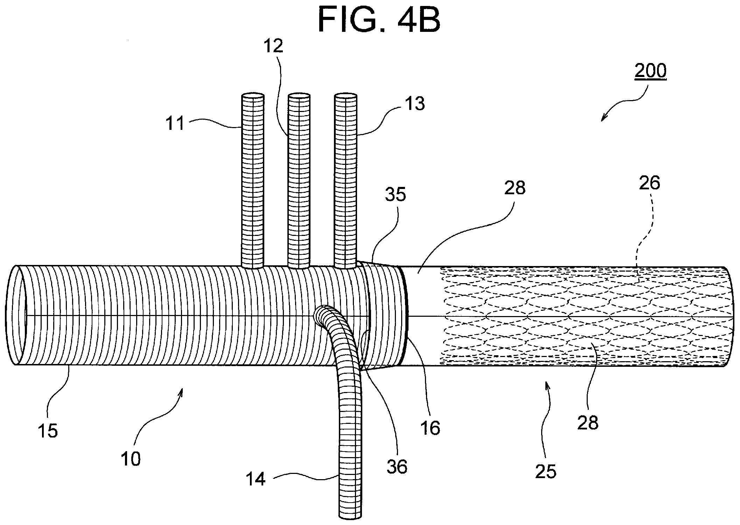

In the present embodiment illustrated in Fig. 4 (Fig. 4A and Fig. 4B), components that are the same as those of the first embodiment will be denoted the same numerals as in Fig. 1 (Fig. 1A and Fig. 1B), and the description thereof will be omitted.

An aorta treatment device 200 according to the present embodiment, which is illustrated in Fig. 4 (Fig. 4A and Fig. 4B), includes: a branched artificial blood vessel portion 10 including four side tubes 11 to 14 that branch off from a main tube 15; a stent graft portion 25 coupled to a distal side of the branched artificial blood vessel portion 10 by being sutured to a distal end 16 of the branched artificial blood vessel portion 10; and an evertable cuff portion 35 that is formed so as to extend from the distal end 16 of the branched artificial blood vessel portion 10 toward the distal side (see Fig. 4A), and that enters a state in which the cuff portion 35 extends from the distal end 16 of the branched artificial blood vessel portion 10 toward the proximal side by being everted (see Fig. 4B).

The stent graft portion 25 of the treatment device 200 according to the present embodiment includes a selfexpandable stent 26 that can be held in a reduced-diameter state and a graft 28 that covers an outer periphery of the stent 26.

Although the stent graft portion 25 is illustrated in an increased-diameter state in Fig. 4 (Fig. 4A and Fig. 4B), until the diameter is increased in a living blood vessel, the stent graft portion 25 is attached to a delivery shaft (not shown) in a reduced-diameter state.

The length of the stent graft portion 25 is the same as that of the stent graft portion 20 according to the first embodiment.

The diameter of the stent graft portion 25 is substantially the same as the diameter of the main tube 15 of the branched artificial blood vessel portion 10 (the ratio of the inside diameter of the stent graft portion 25 to the inside diameter of the main tube 15 is 1.0).

The structure, the material, and the like of the stent 26 and the graft 28 of the stent graft portion 25 are similar to those of the stent 21 and the graft 23 of the stent graft portion 20 according to the first embodiment.

The cuff portion 35 of the treatment device 200 according to the present embodiment is formed from a material (knit fabric) that is the same as the material of the branched artificial blood vessel portion 10 so as to be continuous with the distal end 16 of the branched artificial blood vessel portion 10.

That is, the branched artificial blood vessel portion 10 and the cuff portion 35 are formed from one tubular knit fabric. Moreover, the stent graft portion 25 is coupled to the branched artificial blood vessel portion 10 by being sutured to the distal end 16 of the branched artificial blood vessel portion 10.

Thus, the treatment device 100 has high integrity between the branched artificial blood vessel portion 10 and the cuff portion 35, and can reliably avoid leakage of blood from a space between the branched artificial blood vessel portion 10 and the cuff portion 35.

As illustrated in Fig. 4A, in a non-everted state, the cuff portion 35 of the treatment device 200 extends from the distal end 16 of the branched artificial blood vessel portion 10 toward the distal side so as to cover an outer periphery of a proximal end part of the stent graft portion 25, and an open end 36 of the cuff portion 35 is positioned on the distal side of the distal end 16 of the branched artificial blood vessel portion 10.

The cuff portion 35 has a substantially cylindrical shape such that the inside diameter of the base end thereof (the distal end 16 of the branched artificial blood vessel portion 10) is substantially equal to the inside diameter of the open end 36 (the inside diameter of the open end 36 is slightly larger).

The length of the cuff portion 35 is 5 tc 30 mm, and a preferable example is 15 mm.

The ratio of the inside diameter of the open end 36 to the inside diameter of the base end is lower than 1.5, and a preferable example is 1.02.

The cuff portion 35 can be everted so as to be turned inside out (so that an inner periphery and an outer periphery are reversed).

Fig. 4B illustrates a state in which the cuff portion 35 is everted, and the cuff portion 35 extends from the distal end 16 of the branched artificial blood vessel portion 10 toward the proximal side, and the open end 36 of the cuff portion 35 is positioned on the proximal side of the distal end 16 of the branched artificial blood vessel portion 10.

The cuff portion 35 is treated with a coating of collagen, gelatin, or the like, and thus leakage of blood from the cuff portion 35 can be prevented.

The treatment device 200 according to the present embodiment can be attached by using a delivery system (not shown) in a way similar to that of the treatment device 100 according to the first embodiment.

With the treatment device 200 according to the present embodiment, because the proximal end part of the distal aorta and the cuff portion 35 can be disposed in substantially coaxial cylindrical shapes when suturing these, it is not necessary to perform a cumbersome operation, such as adjustment of the size of a collar, which has been performed when using an existing treatment device, these can be easily sutured, and tension does not remain after suturing.

Heretofore, treatment devices according to the disclosure may be modified in various ways.

For example, the number of side tubes that branch off from the main tube of the branched artificial blood vessel portion need not be four, and may be one, two, or three. Moreover, the disposition of branches may be changed as appropriate.

Although ribs may be formed in the graft of the stent graft portion, the forming density of the ribs may be lower than that of the branched artificial blood vessel portion.

The branched artificial blood vessel portion and the cuff portion need not be formed from one tubular knit fabric, and, for example, the cuff portion may be sutured to the distal end of the tubular knit fabric of the branched artificial blood vessel portion. Ribs may be formed in the cuff portion.

The ratio of the inside diameter of the stent graft portion to the inside diameter of the main tube of the branched artificial blood vessel portion may be lower than 1.0 (for example, 0.8 or higher and lower than 1.0). Reference Signs List

- 100: aorta treatment device
- 10: branched artificial blood vessel portion
- 11 to 14: side tubes
- 15: main tube
- 16: distal end of branched artificial blood vessel portion
- 20: stent graft portion
- 21: stent
- 23: graft
- 30: cuff portion
- 31: open end of cuff portion
- 200: aorta treatment device
- 25: stent graft portion
- 26: stent
- 28: graft
- 35: cuff portion
- 36: open end of cuff portion
- 40: suture thread

## Claims

1. An aorta treatment device (100, 200) comprising:
a branched artificial blood vessel portion (10) including a main tube (15) and at least one side tube (11, 12, 13, 14) that branches off from the main tube (15);
a stent graft portion (20, 25) coupled to a distal side of the branched artificial blood vessel portion (10); and
a cuff portion (30) that is formed so as to extend from a distal end of the branched artificial blood vessel portion (10) toward the distal side, that is evertable so as to extend from the distal end of the branched artificial blood vessel portion (10) toward a proximal side, and that has a cylindrical shape or a skirt-like shape, wherein the cuff portion (30) has a skirt-like shape such that a length thereof is 5 to 30 mm and a ratio of an inside diameter of an open end to an inside diameter of a base end is 1.05 to 1.3.

2. The aorta treatment device (100, 200) according to Claim 1, wherein the branched artificial blood vessel portion (10) includes four side tubes (11, 12, 13, 14) that branch off from the main tube (15).

3. The aorta treatment device (100, 200) according to Claim 1 or 2, wherein the cuff portion (30) is formed from a material that is the same as a material of the branched artificial blood vessel portion (10) so as to be continuous with the distal end of the branched artificial blood vessel portion (10), and wherein the stent graft portion (30) is coupled to the branched artificial blood vessel portion (10) by being sutured to the distal end of the branched artificial blood vessel portion (10).

4. The aorta treatment device (100, 200) according to any one of Claims 1 to 3, wherein a ratio of an inside diameter of the stent graft portion (20, 25) to an inside diameter of the main tube (15) of the branched artificial blood vessel portion (10) is 1.01 to 1.5.

5. The aorta treatment device (100, 200) according to any one of Claims 1 to 4, wherein the branched artificial blood vessel portion (10) and the cuff portion (30) are treated with a coating for preventing leakage of blood, and the stent graft portion (20, 25) is not treated with the coating.

## Patentansprüche

1. Aortenbehandlungsvorrichtung (100, 200), umfassend:
einen verzweigten künstlichen Blutgefäßabschnitt (10), der einen Hauptschlauch (15) und mindestens einen Seitenschlauch (11, 12, 13, 14), der von dem Hauptschlauch (15) abzweigt, einschließt;
einen Stentgraftabschnitt (20, 25), der mit einer distalen Seite des verzweigten künstlichen Blutgefäßabschnitts (10) gekoppelt ist; und
einen Manschettenabschnitt (30), der ausgebildet ist, um sich von einem distalen Ende des verzweigten künstlichen Blutgefäßabschnitts (10) zu der distalen Seite zu erstrecken, der umstülpbar ist, um sich von dem distalen Ende des verzweigten künstlichen Blutgefäßabschnitts (10) zu einer proximalen Seite zu erstrecken, und der eine zylindrische Form oder eine schürzenartige Form aufweist, wobei der Manschettenabschnitt (30) eine schürzenartige Form aufweist, derart, dass eine Länge davon 5 bis 30 mm beträgt und ein Verhältnis eines Innendurchmessers eines offenen Endes zu einem Innendurchmesser eines Basisendes 1,05 bis 1,3 beträgt.

2. Aortenbehandlungsvorrichtung (100, 200) nach Anspruch 1, wobei der verzweigte künstliche Blutgefäßabschnitt (10) vier Seitenschläuche (11, 12, 13, 14), die von dem Hauptschlauch (15) abzweigen, einschließt.

3. Aortenbehandlungsvorrichtung (100, 200) nach Anspruch 1 oder 2, wobei der Manschettenabschnitt (30) aus einem Material ausgebildet ist, das dasselbe wie ein Material des verzweigten künstlichen Blutgefäßabschnitts (10) ist, um mit dem distalen Ende des verzweigten künstlichen Blutgefäßabschnitts (10) durchgehend zu sein, und wobei der Stentgraftabschnitt (30) mit dem verzweigten künstlichen Blutgefäßabschnitt (10) gekoppelt ist, indem er an das distale Ende des verzweigten künstlichen Blutgefäßabschnitts (10) vernäht wird.

4. Aortenbehandlungsvorrichtung (100, 200) nach einem der Ansprüche 1 bis 3, wobei ein Verhältnis eines Innendurchmessers des Stentgraftabschnitts (20, 25) zu einem Innendurchmesser des Hauptschlauchs (15) des verzweigten künstlichen Blutgefäßabschnitts (10) 1,01 bis 1,5 beträgt.

5. Aortenbehandlungsvorrichtung (100, 200) nach einem der Ansprüche 1 bis 4, wobei der verzweigte künstliche Blutgefäßabschnitt (10) und der Manschettenabschnitt (30) mit einer Beschichtung zum Verhindern eines Austritts von Blut behandelt werden und der Stentgraftabschnitt (20, 25) nicht mit der Beschichtung behandelt wird.

## Revendications

1. Dispositif de traitement aortique (100, 200) comprenant :
une partie vaisseau sanguin artificiel ramifié (10) comprenant un tube principal (15) et au moins un tube latéral (11, 12, 13, 14) qui se ramifie à partir du tube principal (15) ;
une partie greffon d'endoprothèse (20, 25) accouplée à un côté distal de la partie vaisseau sanguin artificiel ramifié (10) ; et
une partie manchon (30) qui est formée de manière à s'étendre à partir d'une extrémité distale de la partie vaisseau sanguin artificiel ramifié (10) vers le côté distal, qui est retournable de manière à s'étendre à partir de l'extrémité distale de la partie vaisseau sanguin artificiel ramifié (10) vers un côté proximal, et qui a une forme cylindrique ou une forme de jupe, dans lequel la partie manchon (30) a une forme de jupe de telle sorte qu'une longueur de celle-ci soit de 5 à 30 mm et qu'un rapport d'un diamètre intérieur d'une extrémité ouverte à un diamètre intérieur d'une extrémité de base soit de 1,05 à 1,3.

2. Dispositif de traitement aortique (100, 200) selon la revendication 1, dans lequel la partie vaisseau sanguin artificiel ramifié (10) comprend quatre tubes latéraux (11, 12, 13, 14) qui se ramifient à partir du tube principal (15).

3. Dispositif de traitement aortique (100, 200) selon la revendication 1 ou 2, dans lequel la partie manchon (30) est formée d'un matériau qui est le même qu'un matériau de la partie vaisseau sanguin artificiel ramifié (10) de manière à être continue avec l'extrémité distale de la partie vaisseau sanguin artificiel ramifié (10), et dans lequel la partie greffon d'endoprothèse (30) est accouplée à la partie vaisseau sanguin artificiel ramifié (10) en étant suturée à l'extrémité distale de la partie vaisseau sanguin artificiel ramifié (10).

4. Dispositif de traitement aortique (100, 200) selon l'une quelconque des revendications 1 à 3, dans lequel un rapport d'un diamètre intérieur de la partie greffon d'endoprothèse (20, 25) à un diamètre intérieur du tube principal (15) de la partie vaisseau sanguin artificiel ramifié (10) est de 1,01 à 1,5.

5. Dispositif de traitement aortique (100, 200) selon l'une quelconque des revendications 1 à 4, dans lequel la partie vaisseau sanguin artificiel ramifié (10) et la partie manchon (30) sont traitées avec un revêtement pour empêcher une fuite de sang, et la partie greffon d'endoprothèse (20, 25) n'est pas traitée avec le revêtement.
